Europäisches Patentamt

European Patent Office

Office européen des brevets

(19)

(11) Veröffentlichungsnummer: **0 268 135 B1**

(12) **EUROPÄISCHE PATENTSCHRIFT**

(45) Veröffentlichungstag der Patentschrift: **16.12.92**

(21) Anmeldenummer: **87116136.0**

(22) Anmeldetag: **03.11.87**

(51) Int. Cl.⁵: **A61K 31/71**, //(A61K31/71, 31:44,31:47,31:505,31:235)

(54) **Coccidiozide Mittel.**

(30) Priorität: **11.11.86 DE 3638446**

(43) Veröffentlichungstag der Anmeldung:
**25.05.88 Patentblatt 88/21**

(45) Bekanntmachung des Hinweises auf die
Patenterteilung:
**16.12.92 Patentblatt 92/51**

(84) Benannte Vertragsstaaten:
**AT BE CH DE ES FR GB IT LI NL**

(56) Entgegenhaltungen:
**EP-A- 0 015 110**

**BIOLOGICAL ABSTRACTS/RRM, Nr. 32076110;
W. RAETHER et al.: "The anticoccidial efficacy of mixtures of salinomycin-sodium or halofuginone with various polyether antibiotics
or synthetic drugs in chickens", & RESE-
ARCH IN AVIAN COCCIDIOSIS PROCEE-
DINGS OF THE GEORGIA COCCIDIOSIS CON-
FERENCE, GAINESVILLE, GA., USA, Nov.
18-20, 1985**

(73) Patentinhaber: **HOECHST AKTIENGESELL-
SCHAFT
Postfach 80 03 20
W-6230 Frankfurt am Main 80(DE)**

(72) Erfinder: **Raether, Wolfgang, Dr.
Falkensteinstrasse 6
W-6072 Dreieich(DE)**

BIOLOGICAL ABSTRACTS/RRM, Nr. 29090356; R.J. GRANT et al.: "The effect on broiler growth and coccidiosis parameters of feeding halofuginone and monensin continuously in the same commercial pens for 2 years", & POULTRY SCIENCE (US) 1985, Vol. 64, no. suppl. 1, p.107

BIOLOGICAL ABSTRACTS/RRM, Nr. 28046378; S. LESSON et al.: "Effect of various anticoccidial combinations on chicken broiler performance", & CANADIAN JOURNAL OF ANIMAL SCIENCE (Canada) 1984, vol. 64, no. 3, p. 795-797

BIOLOGICAL ABSTRACTS/RRM, Nr. 11065668; R.N. BREWER: "Evaluation of various combinations of anti coccidials in floor pens", & POULTRY SCIENCE(USA) 1974 vol. 53, no. 5 p. 1904

## Beschreibung

Monensin ist als Polyetherantibioticum aus US-PS 3,501,568 bzw. Antimicrob. Ag. Chemother. 1967, 349 bekannt, s. auch Merck Index 10. Aufl., S. 893 (1983). Dort wird auch deren Verwendung als Anticoccidiosemittel beschrieben. Die Verbindung wird durch Fermentation hergestellt, s. Fermentation Advances, Pap. Int. Ferment. Symp. 3rd, 1968, D. Perlman, Ed. (Academic Press, N.Y., 1969), S. 517 - 540.

Aus EP-A-0015110 sind coccidiozide Mittel, die Monensin und Carbanilide, wie Nicarbazin enthalten, bekannt. Biological Abstract/RRM, Zusammenfassung Nr. 32076110 beschreibt die Kombination von Monensin mit Halofuginon.

Es wurde nun gefunden, daß die Wirkung von Monensin bei dessen Kombination mit anderen bekannten coccidioziden Wirkstoffen über das zu erwartende Maß hinaus erhöht wird.

Gegenstand der Erfindung sind daher coccidiozide Mittel, die gekennzeichnet sind durch einen Gehalt an Monensin oder dessen physiologisch verträglichem Salz in Kombination mit einem oder mehreren Wirkstoffen der Gruppe Methyl-Benzoquat, Amprolium oder Beclotiamine oder dessen Salz.

Insbesondere kommen Zweierkombinationen in Betracht. Erfindungsgemäß können auch Dreierkombinationen unter Einbeziehung von anderen Wirkstoffen verwendet werden; von den letzten ist bevorzugt die Kombination von Monensin mit Methyl-Benzoquat und Meticlorpindol oder mit Amprolium und Ethopabate.

Die erfindungsgemäß zu verwendenden Kombinationspartner für Monensin sind in der Veterinärmedizin seit längerer Zeit bekannt. Sie werden alle im Merck Index, 10. Aufl., veröff. durch Merck & Co., Inc. USA (1983) beschrieben: Meticlorpindol (3,5-Dichlor-2,6-dimethyl-4-pyridinol) auf S. 341; Methyl-Benzoquat (Nequinate; 3-Methoxycarbonyl-6-n-butyl-7-benzyloxy-4-oxochinolin) auf S. 928; Ethopabate (4-Acetamido-2-ethoxy-benzoesäuremethylester) S. 544; Amprolium (1-[(4-Amino-2-propyl-5-pyrimidinyl)methyl]-2-methyl-piperidinium-chlorid) S. 613 und Beclotiamine (3-[(4-Amino-2-methyl-5-pyrimidinyl)methyl]-5-2-chlorethyl)-4-methyl-thiazolium-chlorid) S. 144. Amprolium oder Beclotiamine können in Form ihrer Hydrochlorid-Additionssalze eingesetzt werden. Anstelle von Beclotiamine kann auch dessen entsprechendes Naphthalin-1,5-disulfonsäuresalz eingesetzt werden.

Die Kombination von Meticlorpindol und Methyl Benzoquat ist als Handelsprodukt [R]Lerbek (Fa. ICI) bekannt. Die Kombination von Amprolium mit Ethopabate ist als Handelsprodukt [R]Amprol Mix Super auf dem Markt (Merck, Sharp & Dohme).

Monensin kann als freie Säure oder als Salz, insbesondere als Alkali-(Na, K)-salz oder Ammoniumsalz eingesetzt werden; bevorzugt wird das Na-Salz verwendet. Das Produkt kann in gereinigter Form oder als Mycel oder Rohprodukt eingesetzt werden.

Die erfindungsgemäßen Kombinationen zeigen synergistische Effekte bei der Bekämpfung der Coccidiose, insbesondere der Geflügelcoccidiose.

Bei der Massenhaltung von Geflügel auf engem Raum, z.B. Geflügelmast oder Geflügelaufzucht, besteht stets die potentielle Gefahr einer durch Eimeria-Spezies verursachten Coccidieninfektion, die ohne chemotherapeutische Bekämpfung zu ernsten wirtschaftlichen Verlusten führt. Coccidieninfektionen verursachen in der Regel Gewichtsdepression und blutige Kotausscheidungen, die als Folge von Läsionen in der Darmschleimhaut auftreten. Schwere Coccidieninfektionen führen beim Geflügel in der Regel auch zu hoher Mortalität.

Durch die Anwendung der erfindungsgemäßen Kombinationen ergeben sich im Vergleich zu den entsprechenden Einzelwirkstoffen erhebliche Vorteile, da geringere Mengen an coccidioziden Mitteln eingesetzt werden können. Hieraus resultieren eine Reduzierung der toxischen Nebenwirkungen der Präparate im Vergleich zur Applikation der Einzelwirkstoffe, eine erhöhte Wirtschaftlichkeit sowie Verringerung der Rückstände in den eßbaren Geweben des Geflügels.

In den erfindungsgemäßen Mitteln können die Gewichtsverhältnisse der Wirkstoffe in weiten Grenzen variieren um synergistische Effekte zu erzielen. Sie liegen bei Kombinationen von Monensin

a) mit Methyl-Benzoquat vorzugsweise zwischen 125:1 bis 1:2, insbesondere zwischen 70:1 bis 8:1

b) mit einem Gemisch von Meticlorpindol und Methyl-Benzoquat vorzugsweise zwischen 25:1 bis 1:5, insbesondere 11:1 bis 1:1

c) mit Amprolium vorzugsweise zwischen 1:1 und 1:6

d) mit Beclotiamine vorzugsweise zwischen 3:1 und 1:5

e) mit einem Gemisch von Amprolium und Ethopabate vorzugsweise zwischen 4:1 und 1:4

(Jeweils Verhältnis Monensin zum Kombinationspartner)

Das Verhältnis von Meticlorpindol zu Methyl-Benzoquat kann im Falle b) vorzugsweise zwischen 20:1 bis 7:1 variieren, während das Verhältnis Amprolium zu Ethopabate im Falle e) vorzugsweise zwischen 30:1 und 10:1 variiert.

Die erfindungsgemäßen Kombinationen eignen sich ganz allgemein zum Schutz von Geflügel, d.h. zur Behandlung von landwirtschaftlichem Nutzgeflügel, wie Hühner, Truthühner, Enten oder Gänsen oder auch anderen Vögeln, wie z.B. Fasanen, Wachteln oder Perlhühnern; letztere Vogelarten werden neuerdings auch in Farmen zur wirtschaftlichen Nutzung gehalten und ebenso wie Hühner häufig und massiv von Coccidien befallen.

Zum erfolgreichen Schutz des Geflügels gegen Coccidiose können die erfindungsgemäßen Mittel zu jeder Zeit eingesetzt werden. Sie können insbesondere in Masthühnerbetrieben oder Aufzuchthäusern von Junghennen Anwendung finden, da dort aufgrund der dauernd im Kot ausgeschiedenen Dauerformen der Coccidien (Oocysten) ein hoher Infektionsdruck für die Geflügelpopulation entsteht. Da unter diesen Bedingungen stets das Risiko eines Coccidioseausbruches besteht, sollten die erfindungsgemäßen coccidioziden Mittel beim Geflügel kontinuierlich und vor Ausbruch einer Coccidiose eingesetzt werden. Die erfindungsgemäßen Mittel können aber auch während kurzer Zeitintervalle, d.h. weniger Tage verabreicht werden.

Der Einsatz der erfindungsgemäßen Mittel und Methoden zur Bekämpfung von Coccidiose wird in üblicher Weise durchgeführt. Entsprechend der Lokalisation der Coccidien im Intestinaltrakt kommt in erster Linie eine orale Applikation infrage. Die erfindungsgemäßen Wirkstoffkombinationen können dabei mit Futtermitteln oder mit Trinkwasser gemischt sein.

Die Wirkstoffkonzentrationen der Kombinationen in den Futtermitteln oder im Trinkwasser können innerhalb bestimmter Grenzen variieren. Sie liegen im allgemeinen zwischen 5 und 300 ppm der Wirkstoffkombination bezogen auf das Futtermittel bzw. Trinkwasser.

Die besonders bevorzugten Konzentrationen für die Bekämpfung der Coccidiose sind im Falle des Futtermittels jeweils 25 bis 125 ppm, insbesondere 40 bis 90 ppm Monensin und

a) 1 bis 30 ppm, insbesondere 1,25 bis 5 ppm Methyl-Benzoquat und

b) 5 bis 120 ppm, insbesondere 8 bis 30 ppm Meticlorpindol/Methyl-Benzoquat

c) 100 bis 150 ppm Amprolium

d) 40 bis 125 ppm Beclotiamine

e) 30 bis 100 ppm Amprolium/Ethopabat.

Im Falle der Verwendung im Trinkwasser wird bevorzugt jeweils ca. die Hälfte der angegebenen Konzentrationen benutzt.

Alle erwähnten Konzentrationen, Verhältnisse, Teile, Mengen oder Prozentangaben sind auf Gewichtseinheiten bezogen.

Die Wirkstoffkonzentrationen der coccidioziden Mittel sind auf Futter- oder Trinkwasserzubereitungen ad libitum bezogen, d.h. zur freien Futter- oder Trinkwasseraufnahme während einer praxisüblichen Mast- oder Aufzuchtperiode. Aufgrund besonderer durch die Praxis bedingter Faktoren kann es sich aber ergeben, daß der Geflügelfachmann diese Anwendungskonzentrationen nach oben anpassen muß, falls das Geflügel mit verschiedenen Futter- oder Wasservorräten versorgt werden muß. Dann enthält aber nur ein Teil der Futter- oder Wasservorräte die erfindungsgemäßen Mittel.

Als Träger für die erfindungsgemäßen synergistisch wirksamen coccidioziden Mittel eignen sich alle in der Geflügelindustrie üblichen Futterformulierungen. Die im folgenden angegebenen Formulierungen für Geflügelfutter sind Beispiele für praxisübliche Formulierungen. Darüber hinaus lassen sich aber auch andere Futtermittel auf der Basis von irgendwelchen Getreidekörnern verwenden, die Vitaminkonzentrate, Mineralkonzentrate oder sonstige Wirkstoffe und Futterzusätze in beliebiger Konzentration enthalten. Sowohl herkömmliche trockene, mehlige oder pelletierte Futtermittel als auch flüssige Suspensionsfutter unter Einschluß von Futtermitteln, wie Destillationsschlempen und Milchnebenprodukten, können bei den erfindungsgemäßen Mitteln verwendet werden.

Für die Herstellung des erfindungsgemäßen Geflügelfutters setzt man gewöhnlich zuerst ein konzentriertes Vorgemisch an, das die synergistisch wirksamen coccidioziden Mittel in hoher Konzentration, beispielsweise von 0,2 bis 75 %, enthält. Zu diesem Zweck werden die coccidioziden Mittel mit physiologisch unbedenklichen Trägern, wie Propylenglykolen, Polyethylenglykolen, inerten Ölen, wie Pflanzenölen, hochraffinierten Mineralölen, Äthanol, Wasser, wässrigen Alkoholen, entweder dispergiert oder in inerte Trägerstoffe eingemischt, wie Vermikulit, Diatomeenerde, Attapulgit, Kalziumkarbonat, Bolus alba. Ebenso eignen sich hierfür organische Trägermaterialien, wie Weizenkleie, Maisschrot, Sojabohnenmehl, Luzernmehl, Reishülsen oder gemahlene Maiskolben und beliebig andere organische Trägerstoffe aus pflanzlichen Produkten.

Die erfindungsgemäßen synergistisch wirksamen Mittel lassen sich ferner auch zusammen mit dem Trinkwasser an Geflügel verabreichen. Ihre Einarbeitung in das Trinkwasser wird durch Zusatz einer wasserlöslichen oder in Wasser suspendierbaren Form der jeweiligen Mittel zu dem Trinkwasser in geeigneter Menge erreicht. Die Herstellung solcher Zubereitungen erfolgt im allgemeinen durch Auswahl einer wasserlöslichen Form der Mittel. Sofern diese nicht gewünscht oder nicht herstellbar sind, können

EP 0 268 135 B1

auch wasserunlösliche Formen, beispielsweise Suspensionen Verwendung finden. Zur Herstellung der Zubereitungen verwendet man physiologisch unbedenkliche Hilfsmittel, durch die die erfindungsgemäßen coccidioziden Mittel in Wasser über längere Zeit in Suspensionen gehalten werden. Hilfsmittel, die sich hierfür eignen sind Quellmittel, wie Alginate, Gelatine, Carboxymethylcellulose oder Polyvinylpyrrolidon. Die erfindungsgemäßen Mittel können aber auch mit verschiedenen oberflächenaktiven Verbindungen suspendiert werden, wie beispielsweise mit Hilfe von Lecithin, Naphthalinsulfonaten, Alkylbenzolsulfonaten, alkylphenol-Polyethylenoxid-Addukten oder Polyoxyethylensorbitanestern. Üblicherweise stellt man zunächst eine konzentrierte Suspension oder auch eine trockene Formulierung aus den erfindungsgemäßen coccidioziden Mitteln und den Suspendierungsmitteln in bestimmten Mischungsverhältnissen her und verdünnt sie dann mit dem Trinkwasser auf die gewünschte Anwendungskonzentration, oder die Vormischungen werden in geeigneten Konzentrationen mit den in der Praxis üblichen Futtermitteln gemischt. Das so medikierte Trinkwasser bzw. Futter wird dann dem Geflügel entweder zunächst ad libitum oder eine bestimmte Zeit angeboten.

Das erfindungsgemäße Behandlungsverfahren läßt sich auch auf andere Verfahren zur Behandlung und Fütterung von Geflügel erweitern. So können beispielsweise die erfindungsgemäßen Zubereitungen mit anderen Wirkstoffen kombiniert werden, wie z.B. mit wachstumsfördernden Mitteln oder Parasitika, die einerseits synthetische Mittel oder andererseits Fermentationsprodukte im weitesten Sinn darstellen.

Die Erfindung ist zwar im besonderen Maße auf den Schutz des Geflügels gegenüber Coccidieninfektionen gerichtet, ist jedoch auch sinngemäß bei anderen Haus- und Nutztieren, wie beispielsweise anderen Vögeln, Kaninchen, Schweinen und Wiederkäuern anzuwenden.

Die Erfindung wird durch nachfolgende Beispiele erläutert.

A. Beispiele für Tierfutter-Zusammensetzungen

Die folgenden Beispiele beziehen sich auf Tierfutterzusammensetzungen, welche zur Applikation der erfindungsgemäßen Wirkstoffkombination benutzt werden können.

I. Mastfutter für Broiler

Zusammensetzung in Gew.-%

| | |
|---|---|
| Fischmehl (60 - 65 %) | 6,0 |
| Futterhefe | 2,0 |
| Rindertalg | 4,7 |
| Sojaschrot (44 %) | 24,0 |
| Luzernegrünmehl | 1,0 |
| Mais | 44,89 |
| Weizen | 6,35 |
| Weizennachmehl | 8,5 |
| Phosphors. Futterkalk | 1,4 |
| Kohlens. Futterkalk | 0,96 |
| Spurenelementvormischung[*] | 0,04 |
| Viehsalz | 0,09 |
| Methionin DL | 0,07 |
| | 100,0 |

5

a) pro kg Futter werden zugesetzt:

| | | |
|---|---|---|
| Vitamin A | i.E. | 12.000 |
| $D_3$ | i.E. | 1.500 |
| E | mg | 18 |
| $B_1$ | mg | 1,5 |
| $B_2$ | mg | 6 |
| Pantothensäure | mg | 9 |
| Nicotinsäure | mg | 24 |
| Vitamin $B_6$ | mg | 4,5 |
| $B_{12}$ | mcg | 24 |
| $K_3$ | mg | 3 |
| Cholinchlorid | mg | 1.300 |

*b) In 1,0 kg Futter enthalten

| | | |
|---|---|---|
| Mn | mg | 106 |
| Zn | mg | 71 |
| Fe | mg | 44 |
| Cu | mg | 3,56 |
| I | mg | 0,4 |
| Co | mg | 0,16 |

II. Aufzuchtfutter für Küken (0. - 8. Woche)

Zusammensetzung in Gew.-%

| | |
|---|---|
| Fischmehl (60 - 65 %) | 5,0 |
| Luzernegrünmehl | 6,0 |
| Sojaschrot (44 %) | 13,0 |
| Futterhefe | 2,8 |
| Rindertalg | 2,0 |
| Gerste | 6,0 |
| Hafer | 6,0 |
| Mais | 37,0 |
| Weizen | 13,0 |
| Weizenkleie | 7,3 |
| Kohlens. Futterkalk | 1,29 |
| (R) Hostaphos | 0,57 |
| Spurenelementvormischung * | 0,04 |
| | 100,0 |

* s. Beispiel I b); ferner werden , wie in Beispiel I a)
beschrieben, Vitamine zugesetzt.

III. Aufzuchtfutter für Küken (9. - 20. Woche)

Zusammensetzung in Gew.-%

| | |
|---|---|
| Luzernegrünmehl | 6,0 |
| Sojaschrot (44 %) | 10,24 |
| Futterhefe | 1,8 |
| Rindertalg | 2,0 |
| Gerste | 8,0 |
| Hafer | 6,0 |
| Mais | 40,0 |
| Weizen | 15,0 |
| Weizenkleie | 8,3 |
| Kohlens. Futterkalk | 1,53 |
| (R) Hostaphos | 1,02 |
| Spurenelementvormischung * | 0,04 |
| Methionin DL | 0,07 |
| | 100,0 |

* s. Beispiel I b); ferner werden Vitamine zugesetzt,
s. Beispiel I a)

IV. Aufzuchtfutter für Küken (ab 21. Woche)

Zusammensetzung in Gew.-%

| | |
|---|---|
| Fischmehl (60 - 65 %) | 1,5 |
| Sojaschrot (44 %) | 19,31 |
| Rindertalg | 1,0 |
| Hafer | 6,7 |
| Mais | 40,0 |
| Weizen | 18,0 |
| Weizennachmehl | 3,0 |
| Methionin DL | 0,07 |
| Futterpaprika | 0,3 |

| | |
|---|---|
| Kohlens. Futterkalk | 8,16 |
| (R)Hostaphos | 1,91 |
| Spurenelementvormischung * | 0,05 |
| | 100,0 |

Ferner werden Vitamine, wie in Beispiel I a) beschrieben zugegeben.

V. Futter für Puten (Zusammensetzung in Gew.-%)

| | Putenstarter-futter 0.-8. Woche | Putenmast-futter I 9.-12. W. | Putenmast-futter II 13.-16. W. |
|---|---|---|---|
| Fischmehl | 9,0 | 5,0 | 2,0 |
| Futterhefe | 4,5 | 2,91 | 4,0 |
| Fett | | 3,1 | 6,7 |
| Sojaschrot | 26,0 | 23,9 | 23,87 |
| Luzernegrünmehl | 4,6 | 2,0 | 0,95 |
| Gerste | 5,3 | 4,5 | 5,0 |
| Hafer | | 1,0 | 4,45 |
| Mais | 36,04 | 40,02 | 39,90 |
| Weizen | 5,3 | 6,0 | 5,0 |
| Weizennachmehl | 5,95 | 4,0 | 3,8 |
| Weizenkleie | | 4,0 | |
| Phosphors. Futterkalk | 1,58 | 1,30 | 2,71 |
| Kohlens. Futterkalk | 1,35 | 1,29 | 0,96 |
| Spurenelementvormischung * | 0,04 | 0,04 | 0,04 |
| Viehsalz | 0,24 | 0,84 | 0,44 |
| Methionin | 0,1 | 0,1 | 0,18 |
| | 100,0 | 100,0 | 100,0 |

Vitaminmischung pro kg Futter:

| Vitamin A | i.E. | 12.000 | 8.000 | 8.000 |
|---|---|---|---|---|
| $D_3$ | i.E. | 1.500 | 1.000 | 1.000 |
| E | mg | 18 | 12 | 12 |
| $B_1$ | mg | 1,5 | 1 | 1 |
| $B_2$ | mg | 6,0 | 4 | 4 |

| | | | | |
|---|---|---|---|---|
| Pantothensäure | mg | 9,0 | 6 | 6 |
| Nicotinsäure | mg | 24,0 | 16 | 16 |
| Vitamin $B_6$ | mg | 4,5 | 3 | 3 |
| $B_{12}$ | mcg | 24,0 | 16 | 16 |
| $K_3$ | mg | 3,0 | 2 | 2 |
| Cholinchlorid | mg | 1.300 | 1.300 | 1.300 |

\* in einem 1 kg Futter enthalten: 106 mg Mn; 71 mg Zn; 44 mg Fe; 3,56 mg Cu; 0,4 mg I; 0,16 mg Co.

B. Biologische Beispiele

Der coccidiostatische Effekt der vorliegenden Mittel wurde an mit Coccidien infizierten Hühnchen untersucht. Die im folgenden aufgeführten experimentellen Untersuchungen zeigen die Wirksamkeit der verschiedenen erfindungsgemäßen Kombinationen anhand einiger Beispiele. Das Monensin wird hierbei in Form eines Natriumsalzes des nicht aufgetrennten und durch Fermentation hergestellten Mycels eingesetzt.

Zur Bestimmung und Bewertung des coccidiostatischen Effektes der erfindungsgemäßen Mittel wurden bei allen folgenden beschriebenen experimentellen Untersuchungen sogenannte Infektionskontrollen (unbehandelte, infizierte Tiere) und 0-Kontrollen (unbehandelte, nicht infizierte Tiere) verwandt. Die hierfür verwendeten Tiere beiderlei Geschlechts (LSL Hühnchen, Fa. Lohmann, Wallau, BRD) wurden randomisiert und jeweils Gruppen von 16 Tieren zusammengestellt. Nach der gleichen Methode wurden die mit den erfindungsgemäßen Mitteln behandelten und infizierten Gruppen zussammengestellt. Die Tiere, ausgenommen diegenigen der 0-Kontrolle, wurden mit einem virulenten Eimeria tenella-Stamm infiziert, der in 8 Tage alten Hühnchen zu starken (reproduzierbaren) Läsionen der Blinddärme führt. Die coccidioziden Mittel wurden mit dem Futter für Geflügel in ppm-Mengen vermischt; die jeweiligen Konzentrationen sind aus den folgenden Tabellen 1a bis 1d zu ersehen.

Der Grad der durch die Infektion hervorgerufenen pathologisch-anatomischen Veränderungen der Darmschleimhaut in den Blinddärmen wird üblicherweise (Experimental Parasitology Vol. 28, 1970; oder Long, P.L.: The Biology of the Coccidia, 1982, Univ. Park Press) in Form von Schädigungswerten (= Läsionswerten) im folgen "lesion scores" genannt, (Skala von 0 bis 4) ausgedrückt. Am Ende der Untersuchungen wurden die Tiere 5 Tage nach der Infektion geötet und alle auf typische durch Coccidiose verursachte pathologisch-anatomischen Veränderungen untersucht.

Beschreibung und Bewertung der Schädigung (lesion scores): 0 bis 4

0 = Tiere, bei denen keine Läsionen im Intestinaltrakt nachweisbar sind
1 = Tiere mit wenigen, umschriebenen kleinen Läsionen (Petechien) in den Blinddärmen
2 = Tiere mit mehreren, umschriebenen und etwas größeren Läsionen (Petechien) als unter 1 beschrieben
3 = Tiere mit zahlreichen und relativ großflächigen, blutigen Läsionen, die teilweise konfluieren.
4 = Tiere mit großflächigen, blutigen Läsionen, die die gesamte Darmschleimhaut der Blinddärme und auch angrenzende Darmabschnitte (Hüftdarm = Ileum bzw. Mastdarm) erfaßt. Es bietet sich das Bild einer großflächigen hämorrhagischen Enteritis schwersten Grades, die in der Regel zum Tod des Tieres führt.

Bei den unter 1 bis 4 angegebenen Schädigungen wird in zunehmendem Maß dünnflüssiger und blutiger Kot abgesetzt. Die Gewichtsdepression infolge Nahrungsverweigung korreliert ebenso mit der Zunahme der "lesion scores".

Aus den erwähnten Beobachtungen wird die Relevanz der lesion scores für die Beurteilung der coccidioziden Wirkung der erfindungsgemäßen Mittel deutlich.

Die lesion scores werden einmal als Einzelwerte pro Tier innerhalb einer Gruppe, zum anderen als Mittelwerte der entsprechenden Tiergruppe in den Tabelle 1a - 1d aufgeführt.

Behandlung und Infektionen

In allen Versuchen wurden jeweils 16 eine Woche alte LSL-Hühnchen pro Gruppe unter konstanten Raumbedingungen in Drahtkäfigen zu jeweils 4 Tieren pro Drahtkäfig gehalten. Das mit den erfindungsgemäßen Mitteln medikierte Futter wurde vom Tage D-1 (ein Tag vor der Infektion) bis zum Tage D+5 (5 Tage nach der Infektion) ad libitum angeboten. Die Kontrollgruppen (O-Gruppe bzw. Infektionskontrolle) erhielten nicht medikiertes Futter. Die jeweiligen Tiergruppen wurden somit 7 Tage mit dem gleichen Futter gefüttert. Am Tag der Infektion (=DO) wurden je Tier 200.000 sporulierte Oocysten obengenannten E. tenella-Stammes per Schlundsonde appliziert.

Nach Andauung der Oocysten im Dünndarm wurden aus diesen Dauerformen die infktiösen Sporozoiten freigesetzt, die anschließend die Epithelzellen der Darmschleimhaint befallen und sich dort massenhaft durch Formenänderung vermehren. Die aus den Sporozoiten sich entwickelnden zahlreichen Schizonten zerstören durch wiederholte Teilungsvorgänge das Darmepithel. Der Höhepunkt des zerstörenden Effektes der Schizonten auf das Darmepithel ist 5 Tage nach der Infektion erreicht. Deshalb wurden zu diesem Zeitpunkt der oben beschriebenen Weise die "lesion scores" ermittelt.

Der synergistische Effekt der erfindungsgemäßen Mittel (Kombinationen) wird im folgenden in den Tabellen 1a - 1d dargestellt, und zwar einmal der Effekt der Einzelwirkstoffe in der praxisüblichen Anwendungskonzentration und zum anderen der aus verschiedenen Kombinationen der Einzelwirkstoffe resultierende überadditive oder synergistische Effekt. Die "lesion scores" der Infektionskontrollen bzw. der O-Kontrollen (nicht infizierte Tiere) dienen der Abgleichung mit den jeweiligen medikierten und infizierten Tiergruppen.

## Tabelle 1a

Kombinationen von Monensin mit Methylbenzoquat

| Behandlung medikiertes Futter | Konzentration ppm | Schädigung (lesion scores) Einzelwert pro Tier | Summe | Mittelwert pro Gruppe |
|---|---|---|---|---|
| keine Infektionskontrolle | 0 | 2 3 3 3 3 3 3 4<br>4 4 4 4 4 4 4 4 | 56 | 3,5 |
| keine nicht infiz. Kontrolle | 0 | 0 0 0 0 0 0 0 0<br>0 0 0 0 0 0 0 0 | 0 | 0 |
| Monensin-Na | 100 | 0 0 0 0 0 0 0 0<br>1 1 1 1 1 1 2 2 | 10 | 0,6 |
| | 50 | 0 0 1 1 1 3 3 3<br>3 3 3 4 4 4 4 | 40 | 2,5 |
| | 25 | 3 3 3 3 3 3 3 4<br>4 4 4 4 4 4 4 4 | 57 | 3,6 |
| Methylbenzoquat | 10 | 0 0 0 0 0 0 0 0<br>0 0 0 0 0 0 0 0 | 0 | 0 |
| | 2,5 | 0 0 0 0 0 1 1 1<br>1 3 3 4 4 4 4 4 | 30 | 1,9 |
| Monensin-Na + Methylbenzoquat | 50 + 2,5 | 0 0 0 0 0 0 0 0<br>0 0 0 0 0 0 1 3 | 4 | 0,3 |
| | 25 + 2,5 | 0 0 0 0 0 0 0 0<br>1 1 1 1 3 3 3 3 | 16 | 1,0 |

EP 0 268 135 B1

Tabelle 1

Kombinationen von Monensin mit Meticlorpindol/Methylbenzoquat

| Behandlung medikiertes Futter | Konzentration ppm | Schädigung (lesion scores) Einzelwert pro Tier | Summe | Mittelwert pro Gruppe |
|---|---|---|---|---|
| keine Infektionskontrolle | 0 | 2 3 3 3 3 3 4<br>4 4 4 4 4 4 4 4 | 56 | 3,5 |
| keine nicht infiz. Kontrolle | 0 | 0 0 0 0 0 0 0 0<br>0 0 0 0 0 0 0 0 | 0 | 0 |
| Monensin-Na | 100 | 0 0 0 0 0 0 0 0<br>1 1 1 1 1 1 2 2 | 10 | 0,6 |
| | 50 | 0 0 1 1 1 3 3 3<br>3 3 3 3 4 4 4 4 | 40 | 2,5 |
| | 108,35 | 0 0 0 0 0 0 0 0<br>0 0 0 0 0 0 0 0 | 0 | 0 |
| Meticlorpindol/ Methylbenzoquat | 9,5 | 0 0 0 0 1 1 1 1<br>1 3 3 3 4 4 4 4 | 30 | 1,9 |
| | 6,75 | 1 1 1 1 2 2 3 3<br>3 3 4 4 4 4 4 4 | 44 | 2,8 |
| Monensin-Na + Meticlorpindol/ Methylbenzoquat | 50 + 9,5 | 0 0 0 0 0 0 0 0<br>0 0 0 0 0 0 1 3 | 4 | 0,3 |
| | 50 + 6,75 | 0 0 0 0 0 0 0 0<br>0 0 0 0 3 3 3 3 | 12 | 0,8 |

EP 0 268 135 B1

## Tabelle 1 C

### Kombinationen von Monensin mit Amprolium

| Behandlung medikiertes Futter | Konzentration ppm | Schädigung (lesion scores) Einzelwert pro Tier | Summe | Mittelwert pro Gruppe |
|---|---|---|---|---|
| keine Infektionskontrolle | 0 | 2 3 3 3 3 3 3<br>3 3 4 4 4 4 4 | 53 | 3,3 |
| keine nicht infiz. Kontrolle | 0 | 0 0 0 0 0 0 0 0<br>0 0 0 0 0 0 0 0 | 0 | 0 |
| Monensin | 100 | 0 0 0 0 0 0 0 0<br>0 0 0 1 1 3 3 4 | 12 | 0,8 |
| | 50 | 0 1 1 1 3 3 3 3<br>3 3 3 4 4 4 4 4 | 44 | 2,8 |
| Amprolium | 125 | 0 0 0 0 0 0 0 0<br>0 0 0 0 0 0 0 0 | 0 | 0 |
| | 62,5 | 0 0 0 0 0 0 0 0<br>0 0 0 0 1 1 1 1 | 4 | 0,3 |
| | 31,25 | 0 1 1 1 1 1 1 1<br>2 3 3 4 4 4 4 4 | 44 | 2,8 |
| Monensin + Amprolium | 30 + 62,5 | 0 0 0 0 0 0 0 0<br>0 0 0 0 0 0 0 0 | 0 | 0 |
| | 30 + 31,25 | 0 0 0 0 0 0 0 0<br>0 0 0 0 0 0 1 1 | 2 | 0,1 |

EP 0 268 135 B1

Tabelle 1 - α

Kombinationen von Monensin mit Amprolium + Ethopabate

| Behandlung mediklertes Futter | Konzentration ppm | Schädigung (lesion scores) Einzelwert pro Tier | Summe | Mittelwert pro Gruppe |
|---|---|---|---|---|
| keine Infektionskontrolle | 0 | 2 3 3 3 3 4 4 / 4 4 4 4 4 4 4 | 57 | 3,6 |
| keine nicht infiz. Kontrolle | 0 | 0 0 0 0 0 0 0 / 0 0 0 0 0 0 0 | 0 | 0 |
| Monensin | 100 | 0 0 0 0 0 0 0 / 0 0 1 1 1 2 2 | 9 | 0,6 |
| | 50 | 3 3 3 3 3 4 4 / 4 4 4 4 4 4 4 | 58 | 3,6 |
| | 125 | 0 0 0 0 0 0 0 / 0 0 0 0 0 0 0 | 0 | 0 |
| Amprolium + Ethopabate | 62,5 | 0 0 0 0 0 0 0 / 0 0 1 3 3 3 3 | 7 | 0,4 |
| | 31,25 | 0 0 1 3 3 3 3 / 1 3 3 3 3 3 4 | 22 | 1,4 |
| Monensin + Amprolium + Ethopabate | 50 + 62,5 | 0 0 0 0 0 0 0 / 0 0 0 0 0 0 0 | 0 | 0 |
| | 50 + 31,25 | 0 0 0 0 0 0 0 / 0 0 0 0 0 0 0 | 0 | 0 |

**Patentansprüche**

**Patentansprüche für folgende Vertragsstaaten : AT, BE, CH, DE, FR, GB, IT, LI, NL**

1. Coccidiozide Mittel, gekennzeichnet durch einen Gehalt an Monensin oder dessen physiologisch verträglichem Salz in Kombination mit einem oder mehreren Wirkstoffen der Gruppe Methyl-Benzoquat,

14

Amprolium oder Beclotiamine oder dessen Salz.

2. Mittel gemäß Anspruch 1, dadurch gekennzeichnet, daß es Monensin in Form seines physiologisch verträglichen Salzes enthält.

3. Mittel nach Ansprüchen 1 oder 2, dadurch gekennzeichnet, daß es als Salz ein Alkali- oder Erdalkalisalz enthält.

4. Mittel nach einem oder mehreren der Ansprüche 1 bis 3, dadurch gekennzeichnet, daß es Monensin als Na-Salz enthält.

5. Mittel nach Anspruch 1, dadurch gekennzeichnet, daß es Monensin als Mycel oder Rohprodukt enthält.

6. Mittel gemäß einem oder mehreren der Ansprüche 1 bis 5, dadurch gekennzeichnet, daß es Monensin in Kombination mit Meticlorpindol und Methyl-Benzoquat enthält.

7. Mittel gemäß einem oder mehreren der Ansprüche 1 bis 6, dadurch gekennzeichnet, daß es Monensin in Kombination mit Amprolium und Ethnopabate enthält.

8. Mittel gemäß einem oder mehreren Ansprüchen 1 bis 7, dadurch gekennzeichnet, daß es zusätzlich Geflügelfutter oder Trinkwasser enthält.

**Patentansprüche für folgenden Vertragsstaat : ES**

1. Verfahren zur Herstellung eines coccidioziden Mittels, das Monensin oder dessen physiologisch verträgliches Salz in Kombination mit einem oder mehreren Wirkstoffen der Gruppe Methyl-Benzoquat, Amprolium oder Beclotiamine oder dessen Salz enthält, dadurch gekennzeichnet, daß man die Wirkstoffkombintion in geeigneter Form mit einem Futtermittel oder Trinkwasser oder galenischen Hilfs- und Zusatzstoffen mischt und gegebenenfalls in eine für die orale Verabreichung geeignete Applikationsform bringt.

2. Verfahren gemäß Anspruch 1, dadurch gekennzeichnet, daß das Mittel Monensin in Form seines physiologisch verträglichen Salzes enthält.

3. Verfahren nach Ansprüchen 1 oder 2, dadurch gekennzeichnet, daß das Mittel als Salz ein Alkali-oder Erdalkalisalz enthält.

4. Verfahren nach einem oder mehreren der Ansprüche 1 bis 3, dadurch gekennzeichnet, daß das Mittel Monensin als Na-Salz enthält.

5. Verfahren nach Anspruch 1, dadurch gekennzeichnet, daß das Mittel Monensin als Mycel oder Rohprodukt enthält.

6. Verfahren gemäß einem oder mehreren der Ansprüche 1 bis 5, dadurch gekennzeichnet, daß das Mittel Monensin in Kombination mit Meticlorpindol und Methyl Benzoquat enthält.

**Claims**
**Claims for the following Contracting States : AT, BE, CH, DE, FR, GB, IT, LI, NL**

1. A coccidiocidal agent which contains monensin or its physiologically acceptable salt in combination with one or more active substances of the group comprising methyl benzoquate, amprolium or beclotiamine or its salt.

2. An agent as claimed in claim 1, which contains monensin in the form of its physiologically acceptable salt.

3. An agent as claimed in claims 1 or 2, which contains an alkali metal or an alkaline earth metal salt as salt.

**4.** An agent as claimed in one or more of the claims 1 to 3, which contains monensin as Na salt.

**5.** An agent as claimed in claim 1, which contains monensin as mycelium or crude product.

**6.** An agent as claimed in one or more of the claims 1 to 5, which contains monensin in combination with meticlorpindol and methyl benzoquate.

**7.** An agent as claimed in one or more of the claims 1 to 6, which contains monensin in combination with amprolium and ethopabate.

**8.** An agent as claimed in one or more of the claims 1 to 7, which contains additionally poultry feed or drinking water.

**Claims for the following Contracting State : ES**

**1.** A process for preparing a coccidiocidal agent which contains monensin or its physiologically acceptable salt in combination with one or more active substances of the group comprising methyl benzoquate, amprolium or beclotiamine or its salt, which comprises mixing the active substance combinations in a suitable form with a feedstuff or drinking water or pharmaceutical auxiliary substances and additives and, optionally, converting the mixture to an administration form suitable for oral administration.

**2.** The process as claimed in claim 1, wherein the agent contains monensin in the form of its physiologically acceptable salt.

**3.** The process as claimed in claims 1 or 2, wherein the agent contains an alkali metal or an alkaline earth metal salt as salt.

**4.** The process as claimed in one or more of the claims 1 to 3, wherein the agent contains monensin as Na salt.

**5.** The process as claimed in claim 1, wherein the agent contains monensin as mycelium or crude product.

**6.** The process as claimed in one or more of the claims 1 to 5, wherein the agent contains monensin in combination with meticlorpindol and methyl benzoquate.

**Revendications**
**Revendications pour les Etats contractants suivants : AT, BE, CH, DE, FR, GB, IT, LI, NL**

**1.** Agent coccidiocide, caractérisé en ce qu'il contient de la monensine ou son sel physiologiquement tolérable, en combinaison avec une ou plusieurs substances actives choisies dans le groupe formé par le méthyl-benzoquat, l'amprolium ou la béclotiamine, ou leurs sels.

**2.** Agent selon la revendication 1, caractérisé en ce qu'il contient la monensine sous forme de son sel physiologiquement toléable.

**3.** Agent selon la revendication 1 ou 2, caractérisé en ce qu'il contient comme sel un sel alcalin ou alcalino-terreux.

**4.** Agent selon une ou plusieurs des revendications 1 à 3, caractérisé en ce qu'il contient la monensine sous forme du sel de Na

**5.** Agent selon la revendication 1, caractérisé en ce qu'il contient de la monensine sous forme de mycélium ou de produit brut.

**6.** Agent selon une ou plusieurs des revendications 1 à 5, caractérisé en ce qu'il contient de la monensine en combinaison avec du meticlorpindol et du méthyl-benzoquat.

EP 0 268 135 B1

**7.** Agent selon une ou plusieurs des revendications 1 à 6, caractérisé en ce qu'il contient la monensine en combinaison avec l'amprolium et l'éthopabate.

**8.** Agent selon une des revendications 1 à 7, caractérisé en ce qu'il contient en outre, des aliments pour volailles ou de l'eau de boisson.

**Revendications pour l'Etat contractant suivant : ES**

**1.** Procédé pour préparer un agent coccidiocide, qui contient de la monensine ou son sel physiologiquement tolérable, en combinaison avec une ou plusieurs substances actives choisies dans le groupe formé par le méthyl-benzoquat, l'amprolium ou la béciotiamine ou leurs sels, procédé caractérisé en ce qu'on mélange la combinaison des substances actives sous forme convenable, avec un aliment ou avec de l'eau de boisson ou avec des adjuvants et additifs galéniques et l'on met éventuellement sous une forme destinée à l'application et convenant bien pour une administration par voie orale.

**2.** Procédé selon la revendication 1 caractérisé en ce que l'agent contient de la monensine sous forme de son sel physiologiquement tolérable.

**3.** Procédé selon les revendications 1 ou 2, caractérisé en ce que l'agent contient comme sel un sel alcalin ou alcalino-terreux.

**4.** Procédé selon l'une ou plusieurs des revendications 1 à 3, caractérisé en ce que l'agent contient de la monensine sous forme du sel de sodium.

**5.** Procédé selon la revendication 1, caractérisé en ce que l'agent contient de la monensine sous forme de mycélium ou de produit brut.

**6.** Procédé selon une ou plusieurs des revendications 1 à 5, caractérisé en ce que l'agent contient de la monensine en combinaison avec du méticlorpindol et du méthyl-benzoquat.

17